(19) Europäisches Patentamt
European Patent Office
Office européen des brevets

(11) Publication number: **0 008 711**
**B1**

(12) **EUROPEAN PATENT SPECIFICATION**

(45) Date of publication of patent specification: **04.11.81**

(51) Int. Cl.³: **C 07 C 93/06,**
C 07 D 263/20

(21) Application number: **79102949.9**

(22) Date of filing: **13.08.79**

(54) **A method of preparing a 2-hydroxy-3-isopropyl or a 2-hydroxy-3-tert-butylaminopropyl aromatic ether.**

(30) Priority: **15.08.78 US 933923**

(43) Date of publication of application:
**19.03.80 Bulletin 80/6**

(45) Publication of the grant of the European patent:
**04.11.81 Bulletin 81/44**

(84) Designated Contracting States:
**AT BE CH DE FR GB IT LU NL SE**

(56) References cited:
**CH - A - 481 875**
**DE - A1 - 2 538 424**
**DE - A1 - 2 623 314**
**GB - A - 1 307 405**

(73) Proprietor: **SMITHKLINE CORPORATION**
**1500 Spring Garden Street P.O. Box 7929**
**Philadelphia, Pennsylvania 19101 (US)**

(72) Inventor: **Kirkpatrick, David Samuel**
**2625 Summit Avenue**
**Broomall, Pennsylvania 19008 (US)**
Inventor: **Webb, Robert Lee**
**512 N. Veronica Road**
**West Chester, Pennsylvania 19380 (US)**

(74) Representative:
**Vossius.Vossius.Tauchner.Heunemann.Rauh et al,**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 008 711 B1

Courier Press, Leamington Spa, England.

A method of preparing a 2-hydroxy-3-isopropyl or a 2-hydroxy-3-tert-butylaminopropyl aromatic ether

This invention comprises a method and intermediates useful for inserting the traditional β-blocking side chains,

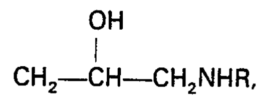

into aromatic nuclei by the use of a 2-oxazolidinone protecting group.

GB A 1 307 405 discloses a process for preparing alkylaminoalcohol derivatives by reacting 5-chloromethyloxazolidines with a phenol and hydrolysing the product obtained. In CH A 481 875 the hydrolysis of N-Isopropyl-5-aryloxy-methyl-2-oxazolidinone derivatives to the respective aromatic ethers with aqueous sodium hydroxide at 120°C is described.

We have unexpectedly discovered that 5-halomethyl-2-oxazolidinones having a 3-isopropyl or 3-tert.-butyl group are difficult to condense with hydroxyaryl compounds in a Williamson-type reaction and also that the resulting aryloxy-methyl-2-oxazolidinones are very stable to many organic reactions useful in elaborating other portions of the molecule. Once introduced the N-isopropyl or tert.-butyl-oxazolidinone ring is difficult to split selectively.

This invention therefore comprises the use of a 5-halomethyl-3-isopropyl or 3-tert.-butyl-2-oxazolidinone to introduce an α-blocking side chain, for example, a 2-hydroxy-3-isopropyl or tert.-butylaminopropyl moiety into an hydroxy aryl nucleus to give an anti-hypertensive compound known to the art. The oxazolidinonemethyl halide is condensed with an aromatic compound having a phenolic hydroxy group in an etherification reaction and then is subsequently split.

The previously unknown starting materials specifically useful for this invention are represented by the following formula:

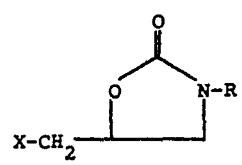

(I)

in which R is isopropyl or *tert.*-butyl and X is bromo, iodo or especially chloro.

The starting materials of Formula I are conveniently prepared by reacting *tert.*-butyl or isopropylisocyanate with an epihalohydrin usually in approximately equimolar quantities in an organic solvent which is inert toward the reactants and in which the reactants are soluble. For example an aromatic solvent such as benzene, toluene or xylene conveniently at reflux temperature may be used. A metallic catalyst such as lithium halide, chloride, bromide or iodide or a quaternary ammonium halide such as tetraethylammonium chloride may also be used. The solvent is not absolutely necessary if the reactants are miscible at the reaction temperature used.

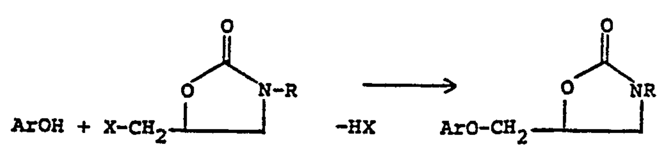


II I III

5-Halomethyl-3-isopropyl or 3-tert.-butyl-2-oxazolidinones (I) react with various aryl hydroxy compounds in a Williamson-type etherification reaction only at elevated temperatures from 110°C to reflux.

Any inert organic solvent in which the reactants are soluble and which has a reflux point within the ranges given can be used. Examples are the higher boiling alcohols such as butanol or propanol, dimethylformamide, dimethylacetamide or dimethylsulfoxide. *n*-Butanol at reflux is a preferred solvent for a proper temperature of reaction as well as for inducing good crystallization of the product of reaction. An acid binding agent must be included for example an alkali metal hydroxide, lower alkoxide or carbonate. Sodium or potassium carbonate is preferred in the optional presence of at least one tenth equivalent of sodium or potassium iodide to facilitate O-alkylation. The term "lower alkoxide" includes alkoxides of 1—6 carbons especially ethoxide or methoxide.

The reaction is most conveniently carried out at reflux temperature until the reaction is substantially complete, usually about 8 to 24 hours.

In the above noted reaction, (I + II = III), R and X are as described above. Ar represents any hydroxy containing aromatic nucleus into which one wishes to introduce the hypotensive side chain. The hydroxy group (—OH) may be characterized as having phenolic properties. Examples of aromatic residues for Ar are optionally substituted or unsubstituted aryl moieties for example phenyl, naphthyl, anthryl, dihydroanthryl, fluorenyl, dihydronaphthyl, tetrahydronaphthyl, indanyl, indenyl, (thio)chromanyl, benzodioxanyl, quinolinyl, isoquinolinyl, benzofuranyl, benzothienyl, benzimidazolyl, thiazolyl, thiadiazolyl or pyridazinylphenyl.

Exemplary of substituents on the aromatic moieties are one or more of the following: halo, 1—10 carbon alkyl, alkoxy or alkylthio, 3 or 4 carbon alkylene, 1—10 carbon alkanoyl, aralkanoyl or aroyl, 1—5 carbon haloalkyl or haloalkoxy, 1—6 carbon alkenyl, alkenyloxy or alkynyloxy, cycloalkoxy, 1—10 carbon aryl, aryloxy, hydrazino, and others.

Preferred aryl moieties are 1-naphthyl, m-tolyl, 2-substituted phenyl, 1-methyl or 1-ethyl- 4 or 5 indolyl or *o*-3-pyridazinylphenyl with a halo or hydrazino substituent at the 6-position.

The desired 5-(aryloxymethyl)-N-*tert.*-butyl or isopropyl-2-oxazolidinones are isolated from the reaction mixture in 15—50% yields using standard chemical methods.

At this point the chemically sensitive β-blocking side chain is masked by the oxazolidinone ring which, generally speaking, is less sensitive to various chemical reactions. Chemical manipulations such as Friedel Crafts reaction, acid catalyzed condensations, nitrations, sulfonation, and other electrophilic aromatic substitution, hydrogenations, hydrogenolysis or reactions which require the use of basic conditions at lower temperatures ($<100°$) do not affect the oxazolidinones but which often destroy the unprotected β-blocking side chain. Thus the oxazolidinone intermediates can be used to effect a large number of chemical transformations on other parts of the molecule with the sensitive β-blocking side chain liberated as the final step.

The N-isopropyl or *tert.*-butyl-5-aryloxymethyl-2-oxazolidinones of Formula III have also been found difficult to split to the desired medicinal agent. For example, attempts to hydrolyze the oxazolidinone ring using acid conditions, for example in 6 N hydrochloric acid at reflux temperature for 18 hours, removed the essential N-alkyl substituent. On the other extreme, alkali in methanol at reflux for 6 hours gives no reaction.

Of course oxazolidinones in general are well known in the art. The most usual chemical means of splitting oxazolidinones in the art is using dilute (0.5—1N) sodium hydroxide or potassium hydroxide in aqueous methanol or ethanol. We have unexpectedly found however that at elevated temperatures of from 100—150°C. until the reaction is complete, alkali metal hydroxides or lower alkoxides of 1—6 carbons such as methoxide or ethoxide split the N-isopropyl or *tert.*-butyl oxazolidinone rings to give the desired β-blocking compound in excellent yields. The reaction is run using, most conveniently, about equimolar quantites of the reactants although some excess of base is acceptable. The reaction medium may be any inert organic solvent in which the reactants are soluble and which is liquid at the critical temperature range. Exemplary are ethylene glycol, propylene glycol, glycol methyl ether, butyl alcohol, isobutylene glycol or polyethylene glycol. In practice the solvent will be water containing. A preferred reaction medium is ethylene glycol — 85% potassium or sodium hydroxide. In this medium a reaction time of about 24 hours at about 120°C gave excellent yield. The product is isolated by standard methods or if a subsequent chemical step is desired may be used without isolation.

The following examples are intended to teach the practice of this invention but not to limit its scope. All temperatures are on the Centigrade scale.

## Example 1

N-(1,1-Dimethylethyl)-5-chloromethyl-2-oxazolidinone

300 G. (3.03 m) of *tert.*-butyl isocyanate, 280.4 g (3.03 m) of epichlorohydrin, 237 ml. of dry toluene, and 1.5 g of lithium chloride were combined and heated to reflux under nitrogen for 24 to 48 hours. The reaction was monitored by gas chromatography to observe the decrease in starting material and increase in product via an unisolated intermediate.

The toluene solvent was removed to obtain crude product which was then recrystallized from cyclohexane; yield: 400 g (69%), m.p. 53.8°.

The title compound has also been prepared by heating the isocyanate and epichlorohydrin with lithium chloride in the absence of solvent. The yields from this method were 63—65%. Here the reaction temperature increased gradually from 90—110° over several hours as starting materials were consumed. At 110°, there was rapid increase in temperature which was hard to control without special equipment. The use of solvent is a means for controlling this exotherm and also for permitting a longer reaction time to ensure completeness with minimal decomposition.

## Example 2

N-(1-Methylethyl)-5-chloromethyl-2-oxazolidinone

50 G. (0.587 m) of isopropyl isocyanate, 54.3 g (0.587 m) of epichlorohydrin, 46 ml. of dry toluene and 1,0 g of lithium chloride were combined and heated at reflux for a total of thirty hours. The reaction was monitored by vapor phase chromatography (VPC). Gas chromatography (GC) at six hours showed 48% oxazolidinone, 45% lower boiling intermediate. At 24 hours, 79% oxaxolidinone, 18%

intermediate (not identified). After 24 hours, the data showed a small decrease in product and intermediate percentages, and the appearance and increase of a third component (lowest boiling). The toluene solvent was removed and the crude reaction mixture was distilled under vacuum. The lower boiling intermediate (unidentified) was also a solid with melting point of about 100°. This solidified in the distillation condenser. The procedure was continued until no further solid was distilled over and the pot showed less than 0.5% low boiling component by VPC. Then a single product cut was taken which was the title compound: yield 51.6 g. (50%) of colorless liquid; b.p. 108—115°/0.15 mm.

The above compound was also formed in a reaction without solvent. The product yield was 43% with 98% purity by VPC.

Example 3

N-(1,1-Dimethylethyl)-5-bromomethyl-2-oxazolidinone

100 G. (1.0 m) of *tert.*-butyl isocyanate, 137 g. (1.0 m) of epibromohydrin, 0.5 g. of lithium bromide (anhydrous) and 85 ml. of toluene were combined and heated to reflux under nitrogen for eight hours. The reaction temperature increased from 100° initially to 135°. VPC showed 62% product present which was constant from four to eight hours. The reaction mixture was cooled and stripped to oil, then the residue was taken up in 800 ml of cyclohexane. The extract was filtered hot and cooled. A solid precipitated which was isolated. An oil separated from the cyclohexane and was distilled. Some decomposition was apparent during distillation. 50 G. (21.5% yield) of the title compound was collected at 119—135°/0.25—0.40 mm. VPC showed 87% purity. This product partially crystallized on standing. It was then recrystallized by taking up in 165 ml. of ether and dilution with 330 ml. of hexane: yield, 31.5 g of the title compound (13.5%); m.p. 38°.

Example 4

N-(1,1-Dimethylethyl-5-(p-chlorophenoxymethyl)-2-oxazolidinone

12.8 G. (0.1 m) of p-chlorophenol was combined with 13.8 g (0.1 m) of anhydrous potassium carbonate in 250 ml. of n-butanol and the mixture heated to reflux for 30 minutes. 1.7 G. (.01M) of potassium iodide was added. Then at reflux, a solution of 19.2 g (0.1 m) of the product of Example 1 in 100 ml. of n-butanol was added over 30 minutes. The reaction was monitored by VPC. After 18 hours at reflux, 45% of product was observed. No increase in the percent product present was seen up to 48 hours at reflux. The reaction mixture was filtered hot to remove inorganic salts, then cooled to crystallize the product which was filtered, washed with isopropanol and dried; yield 12.3 gms (43% of the title compound, m.p. 128°.

The title compound has also been formed using n-butanol with potassium hydroxide (1 m. eq.) plus potassium iodide at reflux — yield 40%.

Example 5

N-(1,1-Dimethylethyl)-5-($\alpha$-naphthyloxymethyl)-2-oxazolidinone

13.8 G. (0.1 m) of potassium carbonate (anhydrous), 19.2 g. (0.1 m) of N-(1,1-dimethylethyl)-5-chloromethyl-2-oxazolidinone, 14.4 g (0.1 m) of $\alpha$-naphthol, 250 ml of n-butanol and 1.7 g of potassium iodide were combined and heated at reflux for 23 hours. The reaction was monitored by VPC. Maximum percent product was 48% at 23 hours. The reaction mixture was filtered hot to remove inorganic salts. The n-butanol was removed under vacuum. The residue obtained was dissolved in 500 ml. of diethyl ether. The extract was filtered through a filter aid, charcoal treated and refiltered. The filtrate was diluted with 500 ml. of petroleum ether to obtain a tacky green solid which was isolated and dissolved in toluene. The toluene extract was separated from an insoluble green oil, charcoal treated cold and then filtered and concentrated. The solid obtained was recrystallized from 10 volumes isopropanol over charcoal to give 5.05 g (17%) of the title compound; m.p. 118—122°.

Example 6

N-(1-Methylethyl)-5-($\alpha$-naphthyloxymethyl)-2-oxazolidinone

14.4 G. (0.1 m) of $\alpha$-naphthol, 13.8 g (0.1 m) of anhydrous potassium carbonate and 250 ml of n-butanol were combined and heated at reflux for 15 minutes. 1.7 G. of potassium iodide was added followed by 17.8 g (0.1 m) of N-(1-methylethyl)-5-chloromethyl-2-oxazolidinone over one hour. The reflux period was extended for 24 hours. The mixture was filtered hot to remove inorganic salts and cooled to precipitate product. The crude product was isolated and recrystallilzed twice from isopropanol over charcoal; yield 8.6 g (30%) of the title compound, m.p. 123—125°.

Example 7

1-(t-Butylamino)-3-(p-chlorophenoxy)-2-propanol

2.0 G. (.007 m) of the product of Example 3 was combined with 25 ml of ethylene glycol and 2.47 g (0.375 m) of 85% potassium hydroxide. The mixture was heated at 120° for 22 hours. The reaction was monitored by TLC; toluene: methanol: ammonium hydroxide — 80:20:1 on silica. The reaction solution was combined with ice water and the precipitated solids were filtered off. The crude product was slurried in 50 ml. of water. 1.0 Ml. of conc. hydrochloric acid was added then the mixture

was filtered to remove undissolved solids (unhydrolyzed oxazolidinone). The filtrate was made basic by addition of 1.2 ml. of 10N sodium hydroxide. The reprecipitated product was filtered, washed well with water, and dried; yield 1.3 g (72%) of the title product, m.p. 118°.

Example 8

1-(t-Butylamino)-3-($\alpha$-naphthyloxy)-2-propanol

2.0 G. (0.0067 m) of N-(1,1-dimethylethyl)-5-(2-naphthyloxymethyl-2-oxazolidinone, 25 ml. of ethylene glycol and 2.47 g. (0.0375 m) of 85% potassium hydroxide were combined and heated at 120° for 24 hours. Water was added to the cooled reaction mixture. A tacky grey-green solid precipitated. The supernatant liquid was poured off through a filter aid. The solids and filter cake were combined and slurried in fresh water. The water was acidified with conc. hydrochloric acid and refiltered. The filtrate was neutralized with 10N sodium hydroxide to precipitate a tacky solid which was filtered through a filter aid. The solids and filter aid were extracted into hot isopropanol. The extracts were filtered. The filtrate was made acid with conc. hydrochloric acid. The alcohol present was removed under vacuum to give the title product; 1.2 g (58%), m.p. 177—179°.

Example 9

1-(Isopropylamino)-3-($\alpha$-naphthyloxy)-2-propanol (propranolol)

2.0 G. (0.007 m) of N-(1-methylethyl)-5-($\alpha$-naphthyloxymethyl)-2-oxazolidinone of Example 6, 25 ml. of ethylene glycol, and 2.47 g (0.0375 m) of 85% potassium hydroxide were combined and heated at 120° for 22 hours. The reaction was then poured into ice water and slurried to precipitate a tacky off-white solid which became crystalline with stirring. The solid was isolated, then combined with fresh water and acidified with 1.0 ml. of conc. hydrochloric acid. After filtration through a filter aid to clarify, the filtrate was neutralized with 10N sodium hydroxide. The precipitate was filtered, washed, and dried to give the title compound; yield 1.5 g (83%) m.p. 92.5°. The base was extracted into 15 ml. of isopropanol with heat. 0.5 Ml. of conc. hydrochloric acid was added. After filtration and cooling, the hydrochloride salt precipitate; 1.5 g, (72.5%) m.p. 162.8°.

Example 10

3 - (3 - t - Butylamino - 2 - hydroxypropoxy) - 4 - morpholino - 1,2,5 - thiadiazole hydrochloride (timolol hydrochloride)

A mixture of 13.7 g (0.1 m) of 3-chloro-4-hydroxy-1,2,5-thiadiazole, 19.2 g (0.1 m) of N-*t*-butyl-5-chloromethyl-2-oxazolidinone, 1.7 g (0.01 m) of potassium iodide and 13.8 g (0.1 m) of anhydrous potassium carbonate in 250 ml. of *n*-butanol was heated at reflux with stirring for 24 hours. The mixture was filtered hot and the filtrate stripped to dryness on a rotary evaporator. The residue was chromatographed on silica gel using chloroform/methanol to give 3-(5-oxymethyl-N-*t*-butyl-2-oxazolidinyl)-4-chloro-1,2,5-thiadiazole as a viscous oil.

2.92 G. (0.01 m) of the oil was heated with 7.6 g (0.1 m) of morpholine at 125° for 4 hours, when 25 ml of ethylene glycol containing 2 g of potassium hydroxide was added and the mixture heated at 120° for a further 24 hours. On cooling the mixture was poured into water and extracted with chloroform. The chloroform extracts were dried and evaporated. Treatment of the residue with ethereal hydrogen chloride gave after purification the title compound m.p. 161°.

**Claims**

1. A method of preparing a 2-hydroxy-3-isopropyl- or a 2-hydroxy-3-tert.-butylaminopropyl aromatic ether comprising:

(A) reacting a 5-halomethyl-3-isopropyl- or 3-tert.-butyl-2-oxazolidinone of the formula:

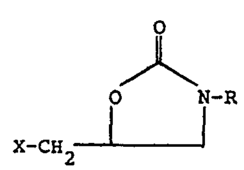

in which R is isopropyl or tert.-butyl and X is bromo, iodo or chloro;

with an aromatic compound containing a phenolic hydroxy group at temperatures from 110°C to reflux in an organic solvent in the presence of an acid binding agent to give a N-isopropyl or tert.-butyl-5-aryloxymethyl-2-oxazolidinone agent; and then

(B) reacting the oxazolidinone with an alkali metal hydroxide or lower alkoxide at a temperature in the range of 100—150°C in an organic solvent.

2. The method of claim 1 in which the organic solvent in the first step of the reaction sequence is n-butanol and the acid binding agent is an alkali metal carbonate.

3. The method of claims 1 or 2 in which the organic solvent in the second step of the reaction

sequence is ethylene glycol in the presence of 85% potassium or sodium hydroxide at a temperature of 100—150°C.

4. The method of claims 1—3 in which R is tert.-butyl.

5. The method of claims 1—4 in which the aromatic compound is $\alpha$-naphthol.

## Revendications

1. Procédé de préparation d'un éther 2-hydroxy-3-isopropyl- ou 2-hydroxy-3-*tert.*-butyl-aminopropyl aromatique caractérisé en ce que:

(A) on fait réagir une 5-halométhyl-3-isopropyl- ou 3-*tert.*-butyl-2-oxazolidinone de formule:

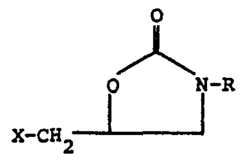

dans laquelle R représente un groupe isopropyle ou *tert.*-butyle et X représente un atome de brome, d'iode ou de chlore;

avec un composé aromatique contenant un groupe hydroxy phénolique, à des températures s'échelonnant de 110°C à la température de reflux, dans un solvant organique, en présence d'un capteur acide, de façon à obtenir un intermédiaire du type N-isopropyl- ou *tert.*-butyl-5-aryloxyméthyl-2-oxazolidinone; et qu'ensuite

(B) on fait réagir l'oxazolidinone avec un hydroxyde de métal alcalin ou un alcoolate inférieur, à une température s'échelonnant de 100 à 150°C, dans un solvant organique.

2. Procédé suivant la revendication 1, caractérisé en ce que le solvant organique utilisé dans la première étape de la séquence réactionnelle est le n-butanol et le capteur acide est un carbonate de métal alcalin.

3. Procédé suivant l'une quelconque des revendications 1 et 2 caractérisé en ce que le solvant organique utilisé dans la seconde étape de la séquence réactionnelle est l'éthylène glycol, en présence d'hydroxyde de potassium ou de sodium à 85%, à une température de 100—150°C.

4. Procédé suivant l'une quelconque des revendications 1 à 3 caractérisé en ce que R représente un groupe *tert.*-butyle.

5. Procédé suivant l'une quelconque des revendications 1 à 4 caractérisé en ce que le composé aromatique est 1'$\alpha$-naphtol.

## Patentansprüche

1. Verfahren zur Herstellung eines 2-Hydroxy-3-isopropyl oder eines 2-Hydroxy-3-tert.-butyl-aminopropyl-aromatischen Äthers, dadurch gekennzeichnet, daß man:

(A) ein 5-Halogenmethyl-3-isopropyl- oder 3-tert.-butyl-2-oxazolidinon der Formel

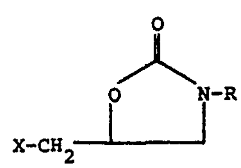

in der R

eine Isopropylgruppe oder einen tert.-Butylrest bedeutet und X ein Brom-, Jod- oder Chloratom darstellt;

mit einer aromatischen Verbindung, die eine phenolische Hydroxylgruppe enthält, bei Temperaturen von 110°C bis zur Rückflußtemperatur in einem organischen Lösungsmittel in Gegenwart eines säurenbindenden Mittels umsetzt, wobei man ein N-Isopropyl- oder tert.-Butyl-5-aryloxy-methyl-2-oxazolidinon-Mittel erhält; und man anschließend

(B) das Oxazolidinon mit einem Alkalimetallhydroxid oder Niederalkoxid bei Temperaturen im Bereich von 100 bis 150°C in einem organischen Lösungsmittel umsetzt.

2. Verfahren nach Anspruch 1, in dem das organische Lösungsmittel in der ersten Stufe der Verfahrensfolge n-Butanol ist und das säurenbindende Mittel ein Alkalimetallcarbonat ist.

3. Verfahren nach den Ansprüchen 1 oder 2, worin das organische Lösungsmittel in der zweiten Stufe der Reaktionsfolge ein Äthylenglykol ist, in Gegenwart von 85prozentigem Kalium- oder Natriumhydroxid und bei Temperaturen von 100 bis 150°C.

4. Verfahren nach den Ansprüchen 1 bis 3, dadurch gekennzeichnet, daß R einen tert.-Butylrest darstellt.

5. Verfahren nach den Ansprüchen 1 bis 4, dadurch gekennzeichnet, daß die aromatische Verbindung $\alpha$-Naphthol ist.